# EUROPEAN PATENT APPLICATION

(11) **EP 4 523 790 A1**
(43) Date of publication of application: **19.03.2025**
(21) Application number: 22941762.1
(22) Date of filing: 15.12.2022
(51) Int. Cl.: B01J 35/02, B01J 27/18, B01J 27/185, B01J 27/199, A61L 9/00, A61L 9/014, A61L 9/20, F24F 8/167, F24F 8/22

(54) **AIR CLEANING DEVICE AND AIR CLEANING METHOD**

(30) Priority: 12.05.2022 JP 2022079040
(71) Applicant: HIDEC CO., LTD., Komaki-shi Aichi 485-0075 (JP)
(72) Inventor: OHKURA Shigenobu, Komaki-shi, Aichi 485-0075 (JP)
(74) Representative: Epping - Hermann - Fischer
(86) International application number: PCT/JP2022/046183
(87) International publication number: WO 2023/218689

(57) **Abstract**

In an air cleaning device (1) including a thin-plate array unit (20) that has a plurality of thin plates (21) arranged parallel to each other with gaps therebetween and that has a coating layer on each of front and rear surfaces of each thin plate (21) and also including an ultraviolet irradiator (35) that radiates ultraviolet light onto the coating layers, each coating layer is a metal-substituted hydroxyapatite layer in which a portion of calcium in calcium hydroxyapatite is substituted by metal selected from titanium, zirconium, iron, and tungsten.

## Description

### Technical Field

The present invention relates to air cleaning devices and air cleaning methods using the air cleaning devices.

### Background Art

With the recent outbreak of the novel coronavirus, there have been measures taken for preventing infections by wearing masks and by setting partitions, such as acrylic boards. It is conceivable that the possibility of being exposed to the virus included in relatively large droplets emitted by coughing and sneezing can be reduced significantly with masks and partitions. However, it is said that minute droplets emitted during breathing and talking float and spread as an aerosol in the air over a long period of time. Although it is recommended that ventilation be performed frequently, ventilation of an entire room is difficult. Therefore, the present inventor has reached a conception that the current measures for preventing infections are insufficient and that there is a need for an air cleaning device that takes in air inside a room, decomposes organic matters, such as viruses, contained in the air, and discharges clean air.

Various air cleaning devices have been proposed in the related art. Of these devices, there is a type of device intended to decompose organic matters contained in the air by having an air filter that carries a photocatalyst and by radiating ultraviolet light thereto (e.g., see Patent Literature 1).

However, since the air travels instantaneously through the filter carrying the photocatalyst, it is extremely difficult in actuality to decompose the organic matters contained in the flowing air in accordance with the function of the photocatalyst.

### Citation List

### Patent Literature

PTL 1: Japanese Unexamined Patent Application Publication No. 2016-128154

### Summary of Invention

### Technical Problem

In view of the circumstances described above, an object of the present invention is to provide an air cleaning device that can decompose organic matters contained in the air more reliably, and to provide an air cleaning method using the air cleaning device.

### Solution to Problem

In order to solve the aforementioned problems, an air cleaning device according to the present invention includes: a thin-plate array unit that has a plurality of thin plates arranged parallel to each other with gaps therebetween and that has a coating layer on each of front and rear surfaces of each thin plate; and an ultraviolet irradiator that radiates ultraviolet light onto the coating layers. Each coating layer is a metal-substituted hydroxyapatite layer in which a portion of calcium in calcium hydroxyapatite is substituted by metal selected from titanium, zirconium, iron, and tungsten.

A photocatalyst, such as titanium oxide or tungsten oxide, does not have a function for adsorbing organic matters. Therefore, even if an air filter carries a photocatalyst, it is extremely difficult in actuality to decompose the organic matters contained in the air traveling instantaneously through the filter in accordance with the function of the photocatalyst.

In contrast, in this configuration, each of the coating layers stacked on the thin plates is a metal-substituted hydroxyapatite layer in which a portion of calcium in calcium hydroxyapatite is substituted by metal selected from titanium, zirconium, iron, and tungsten. The calcium hydroxyapatite portion of the metal-substituted hydroxyapatite has an excellent function for adsorbing organic matters. On the other hand, the portion (referred to as "metal-substituted portion") of the metal-substituted hydroxyapatite in which the calcium is substituted by metal selected from titanium, zirconium, iron, and tungsten has a photocatalytic function.

Therefore, the air cleaning device according to this configuration performs "two-step air cleaning" involving first adsorbing the organic matters from the instantaneously traveling air and setting the organic matters to a non-moving state, and subsequently decomposing the organic matters in accordance with a photocatalytic effect by ultraviolet irradiation. In other words, the air cleaning device according to this configuration is provided with a "time lag" between the adsorption and the decomposition of the organic matters.

Supposing that the metal-substituted hydroxyapatite is carried by a filter, the air would travel through vent holes extending through the filter in the thickness direction. Therefore, even if the metal-substituted hydroxyapatite is carried around the vent holes, the distance by which the flowing air comes into contact with the metal-substituted hydroxyapatite is short, thus possibly resulting in insufficient adsorption of the organic matters. In contrast, in this configuration, the air is caused to flow through the gaps between the thin plates, and the air flows along the front and rear surfaces of the thin plates. This results in planar contact between the air and the coating layers, so that the distance by which the flowing air comes into contact with the coating layers can be extended, whereby the organic matters in the air are effectively adsorbed.

In the air cleaning device according to the present invention, in addition to the above configuration, each of the thin plates may have a shape having a pattern with repetitive recesses and protrusions on the front and rear surfaces, and the recesses and the protrusions may be reflected on each coating layer.

Supposing that the flow of air is a laminar flow, a boundary layer where there is no flow of air occurs between each thin plate having the coating layers and the air layer, possibly resulting in insufficient contact between the air and the coating layers. In contrast, the front and rear surfaces of each thin plate according to this configuration have the pattern with the repetitive recesses and protrusions, so that a turbulent flow occurs in the flowing air. Accordingly, the air flows while changing in various directions, thereby further facilitating the contact between the air and the coating layers.

An air cleaning method according to the present invention includes: causing air to flow through the gaps between the thin plates; and using a calcium hydroxyapatite portion in the metal-substituted hydroxyapatite constituting each coating layer to adsorb organic matters contained in the air and decomposing the adsorbed organic matters in accordance with a photocatalytic function by the portion of the metal-substituted hydroxyapatite in which the calcium is substituted by the metal.

This is an air cleaning method using the air cleaning device according to the above configuration. As mentioned above, this is a "two-step air cleaning" method involving first adsorbing the organic matters from the instantaneously traveling air and setting the organic matters to a non-moving state, and subsequently decomposing the organic matters in accordance with a photocatalytic effect, and a "time lag" is provided between the adsorption and the decomposition of the organic matters.

In addition to the above configuration, the air cleaning method according to the present invention may further include: causing a turbulent flow to occur in the air flowing through the gaps between the thin plates in accordance with the pattern with the repetitive recesses and protrusions to enhance a degree of contact between the air and each coating layer, as compared with when the air is a laminar flow.

This is an air cleaning method using the air cleaning device in which the front and rear surfaces of each thin plate have the pattern with the repetitive recesses and protrusions. The degree of contact between the air and each coating layer is enhanced, so that the organic matters in the air are adsorbed more reliably.

### Advantageous Effects of Invention

Accordingly, the present invention can provide an air cleaning device that can decompose organic matters contained in the air more reliably, and can provide an air cleaning method using the air cleaning device.

### Brief Description of Drawings

[Fig. 1] Fig. 1 schematically illustrates the configuration of an air cleaning device according to a first embodiment of the present invention.
[Fig. 2] Fig. 2 is an exploded perspective view of a relevant part of the air cleaning device in Fig. 1.
[Fig. 3] Fig. 3 is a plan view of a relevant part of the air cleaning device in Fig. 1.
[Fig. 4] Fig. 4 is an exploded perspective view of a relevant part of an air cleaning device according to a second embodiment of the present invention.
[Fig. 5] Fig. 5 is an exploded perspective view of a relevant part of an air cleaning device according to a third embodiment of the present invention.

### Description of Embodiments

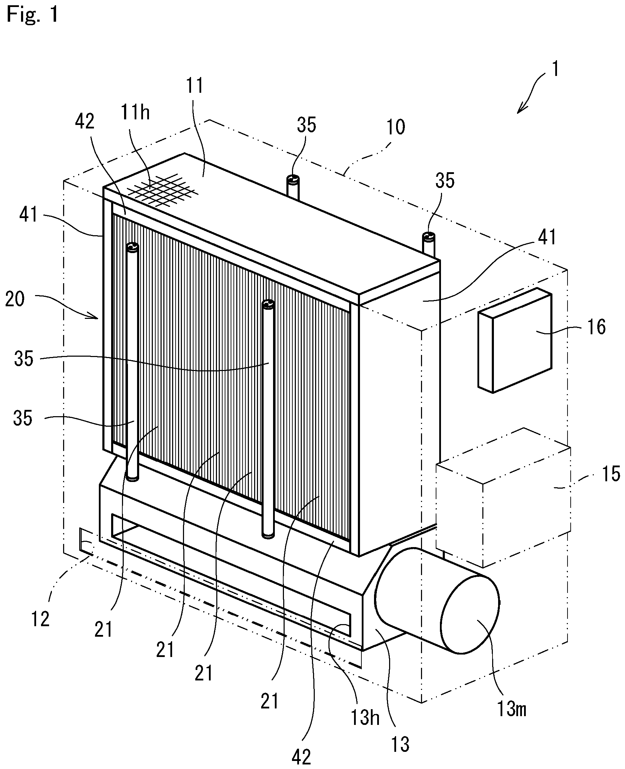

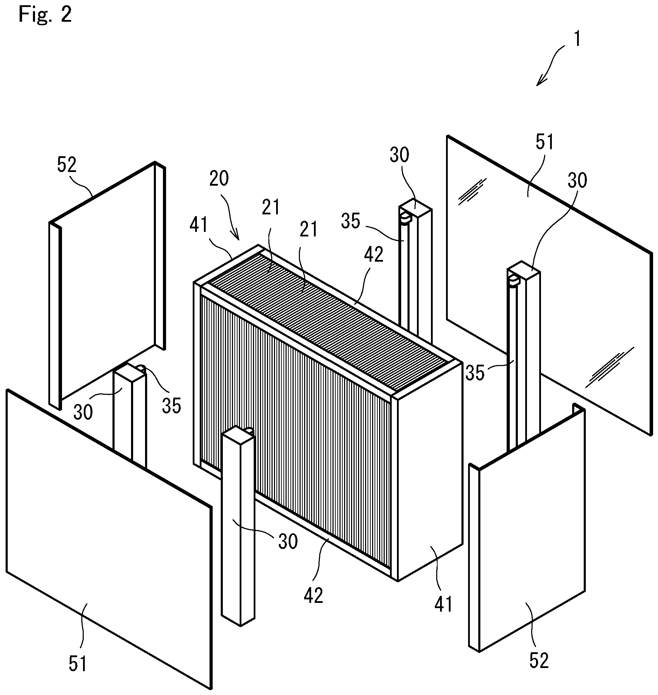

An air cleaning device according to a specific embodiment of the present invention and an air cleaning method using this air cleaning device will be described below with reference to the drawings. First, an air cleaning device 1 according to a first embodiment will be described with reference to Figs. 1 to 3.

The air cleaning device 1 includes a thin-plate array unit 20, ultraviolet irradiators 35, an air blower 13, and a controller 15 that are accommodated in a casing 10 having a substantially rectangular parallelepiped shape. The casing 10 is provided with an inlet 11h and an outlet 12 to communicate with an exterior space of the air cleaning device 1.

The thin-plate array unit 20 includes 200 to 400 thin plates 21 arranged parallel to each other with gaps therebetween. Each thin plate 21 is a rectangular plate member with a small thickness of 0.15 mm to 0.3 mm. Each gap between the thin plates 21 is fixed and is 2.0 mm to 3.0 mmm. Each gap between the thin plates 21 serves as an air flow path.

The thin plates 21 are supported by a support frame so as to be arranged with gaps therebetween. The support frame is formed of a pair of support side surfaces 41 and two pairs of support bars 42. The pair of support side surfaces 41 are parallel to the thin plates 21 and are disposed at opposite outer sides of the arranged thin plates 21. With regard to the pair of support side surfaces 41, the ends thereof at the upstream side of the air flow path are coupled to each other by a pair of support bars 42, and the ends thereof at the downstream side of the air flow path are coupled to each other by a pair of support bars 42.

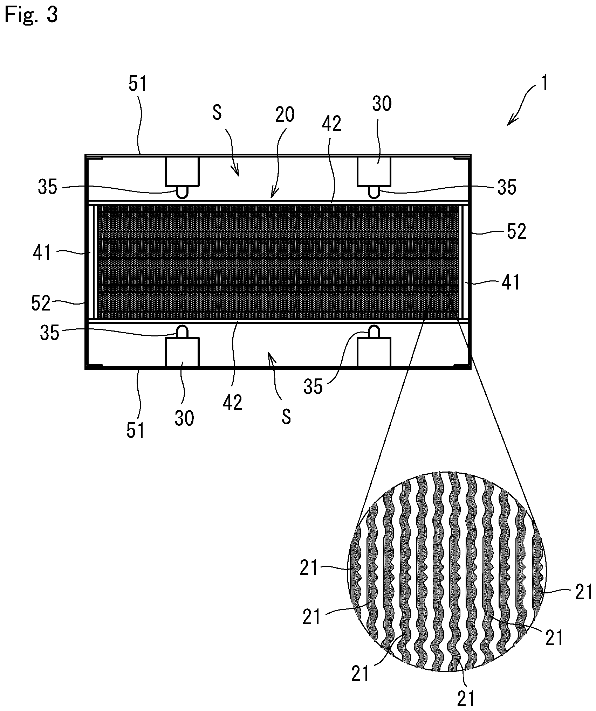

The front and rear surfaces of each thin plate 21 have a pattern with repetitive fine recesses and protrusions (see an enlarged view in Fig. 3). This pattern with repetitive recesses and protrusions can be formed by etching or machinery cutting the thin plate 21.

The front and rear surfaces of each thin plate 21 are each provided with a coating layer (not shown) on the recesses and protrusions. Each coating layer is a metal-substituted hydroxyapatite layer. In metal-substituted hydroxyapatite, a portion of calcium in calcium hydroxyapatite (Ca₁₀(PO₄)₆(OH)₂) is substituted by metal selected from titanium, zirconium, iron, and tungsten. The thin plate 21 is coated with metal-substituted hydroxyapatite by first coating each of the front and rear surfaces of the thin plate 21 with an adhesive and blowing metal-substituted hydroxyapatite powder thereto to adhere the metal-substituted hydroxyapatite thereon, or by spraying or applying a slurry containing a mixture of an adhesive coating agent and metal-substituted hydroxyapatite powder onto the thin plate 21, or by immersing the thin plate 21 in this slurry. Of these methods, the method of adhering the metal-substituted hydroxyapatite powder onto the adhesive preliminarily applied to each of the front and rear surfaces of the thin plate 21 achieves a large exposed surface area in the surface area of the metal-substituted hydroxyapatite particles, and is thus desirable in that an adsorption function and a photocatalytic function can be effectively exhibited. With regard to the metal-substituted hydroxyapatite applied to each of the front and rear surfaces of the thin plate 21, the type of metal substituting for the calcium in the calcium hydroxyapatite may be varied between the front surface and the rear surface.

In the metal-substituted hydroxyapatite, the calcium hydroxyapatite portion has an excellent function for adsorbing organic matters. On the other hand, the metal-substituted portion (i.e., the portion in which the calcium is substituted by metal, such as titanium) in the metal-substituted hydroxyapatite has a photocatalytic function. Specifically, when the metal-substituted portion absorbs light energy equivalent to a band gap between a valence band and a conduction band, an electron in the valence band is excited to the conduction band, and this electron reduces another matter. In the valence band, an electron hole occurs as a result of losing the electron, and the electron hole receives an electron from another matter and oxidizes. Such an oxidation-reduction effect causes organic matters such as viruses, bacteria, and a source for odor to decompose.

The metal-substituted hydroxyapatite can be obtained by mixing an aqueous solution (e.g., a calcium-nitrate aqueous solution or a phosphoric-acid aqueous solution) of the starting material of the calcium hydroxyapatite with an aqueous solution (e.g., a titanium-sulfate aqueous solution if the metal is titanium) containing metal ions substituting for the calcium at a predetermined ratio, subsequently adjusting the pH (potential of hydrogen) to cause precipitation to occur, and then filtering the precipitate.

In this embodiment, air is caused to flow downward from above the gaps between the thin plates 21. Thus, the inlet 11h is provided in the upper surface of the casing 10. The inlet 11h is configured by holes in a net body 11 disposed above the thin-plate array unit 20. The net body 11 also functions as a pre-filter that removes relatively large foreign matters, such as dust and rubbish, from air flowing into the thin-plate array unit 20. The outlet 12 is provided in a lower area of a side surface of the casing 10.

The air blower 13 is provided below the thin-plate array unit 20 and between the thin-plate array unit 20 and the outlet 12, and causes the air from the inlet 11h to flow toward the outlet 12. An inlet (not shown) of the air blower 13 communicates with the downstream end of the thin-plate array unit 20, and an outlet 13h of the air blower 13 faces the outlet 12 provided in the casing 10. The casing 10 also accommodates a motor 13m that rotationally drives blades of the air blower 13.

The controller 15 is a computer that includes a storage device constituted of a main storage unit and an auxiliary storage unit and that also includes a central processing unit. The storage device has stored therein a program for causing the computer to function as air cleaning processing means. The air cleaning processing means mainly performs a process for adjusting the air flow rate by controlling the motor 13m and a process for recording a detection result obtained by a carbon-dioxide concentration sensor 16 together with an operational condition of the air cleaning device 1. The air flow rate is adjusted such that the rate at the outlet 12 is between 5.5 m/s and 6.5 m/s.

There are multiple ultraviolet irradiators 35 provided that are attached, via attachment sections 30, to the inner sides of a pair of first side surfaces 51 facing each other perpendicularly to the pair of support side surfaces 41, thereby radiating ultraviolet light toward the thin-plate array unit 20. The pair of first side surfaces 51 are coupled to each other by a pair of second side surfaces 52. The pair of second side surfaces 52 are secured while being in abutment with the pair of support side surfaces 41, and are wider than the support side surfaces 41. Accordingly, a space S is formed between each of the pair of first side surfaces 51 and the thin-plate array unit 20. The inner surfaces of the pair of first side surfaces 51 are mirror surfaces or mirror-polished surfaces.

Although a state where two ultraviolet irradiators 35 are attached to each first side surface 51 is illustrated, the number of ultraviolet irradiators 35 is not limited to this. Moreover, each ultraviolet irradiator 35 used may be, for example, an ultraviolet irradiation tube that radiates ultraviolet light with a wavelength of 222 nm or 282 nm.

In the air cleaning device 1 having such a configuration, when the air blower 13 is activated, air is taken in through the inlet 11h and flows through the gaps between the thin plates 21 in the thin-plate array unit 20. Because the front and rear surfaces of each thin plate 21 are each coated with a metal-substituted hydroxyapatite coating layer, organic matters contained in the air is adsorbed in accordance with the excellent adsorption function of the calcium hydroxyapatite portion. Specifically, the organic matters are captured from the instantaneously traveling air, so that the organic matters become in a non-moving state.

The distance between the thin plates 21 is extremely short at 2.0 mm to 3.0 mm, so that the gaps between the thin plates 21 serving as air flow paths are extremely narrow. This facilitates contact between the air and each coating layer, so that the organic matters in the air are effectively captured.

Supposing that the flow of air is a laminar flow, a boundary layer where there is no flow of air occurs between each thin plate 21 having the coating layers and the air layer, possibly resulting in insufficient contact between the air and the coating layers. In contrast, the front and rear surfaces of each thin plate 21 according to this embodiment have a pattern with repetitive fine recesses and protrusions, and the recesses and the protrusions are reflected on the coating layers, so that a turbulent flow occurs in the flowing air. Accordingly, the air flows while changing in various directions, thereby further facilitating the contact between the air and the coating layers.

In addition, supposing that the metal-substituted hydroxyapatite is carried by a filter, the air would travel through vent holes extending through the filter in the thickness direction. Therefore, even if the metal-substituted hydroxyapatite is carried around the vent holes, the distance by which the flowing air comes into contact with the metal-substituted hydroxyapatite is short, thus possibly resulting in insufficient capturing of the organic matters. In contrast, in this embodiment, the air is caused to flow through the narrow gaps between the thin plates 21, and the air flows along the front and rear surfaces of the thin plates 21, thus resulting in planar contact between the air and the coating layers. Consequently, the distance by which the flowing air comes into contact with the coating layers can be extended, so that the organic matters in the air can be effectively captured.

Furthermore, in the air cleaning device 1, the inlet 11h is provided in the upper area thereof, and the outlet 12 is provided in the lower area thereof. Relatively large droplets fall downward in a short period of time, whereas an aerosol rises slowly while floating and spreading in the air over a long period of time. Therefore, air containing the aerosols can be readily taken in through the inlet 11h, and the organic matters, such as viruses, contained in the aerosols can be effectively captured by each coating layer.

Accordingly, the air having the organic matters, such as viruses, captured and removed therefrom, that is, clean air, is discharged outward through the outlet 12.

In the air cleaning device 1, ultraviolet light is radiated from the ultraviolet irradiators 35 toward the thin-plate array unit 20. Specifically, the coating layers stacked on the thin plates 21 of the thin-plate array unit 20 are irradiated with the ultraviolet light. Accordingly, as mentioned above, the organic matters captured by each coating layer are decomposed by the photocatalytic function of the metal-substituted portion in the metal-substituted hydroxyapatite constituting the coating layer. Specifically, instead of decomposing the organic matters during the air flowing stage, the air cleaning device 1 performs "two-step air cleaning" involving first capturing the organic matters and setting the organic matters to a non-moving state, and subsequently decomposing the organic matters. In other words, the air cleaning device 1 according to this embodiment is provided with a "time lag" between the adsorption and the decomposition of the organic matters.

Because the inner surfaces of the first side surfaces 51 are mirror surfaces or mirror-polished surfaces, the ultraviolet light radiated from the ultraviolet irradiators 35 is reflected. Accordingly, attenuation of the ultraviolet light after being radiated from the ultraviolet irradiators 35 is reduced, so that the coating layers can be supplied with sufficient light energy, whereby the metal-substituted portion of the metal-substituted hydroxyapatite can sufficiently exhibit the photocatalytic function.

Furthermore, the photocatalytic function of the metal-substituted hydroxyapatite requires light irradiation, whereas the adsorption function of the metal-substituted hydroxyapatite does not require light irradiation. Clean air can be discharged from the outlet 12 so long as the organic matters can be tentatively removed from the air in accordance with adsorption. Thus, the ultraviolet light does not necessarily need to be radiated from the ultraviolet irradiators 35 at all times, and may be radiated intermittently.

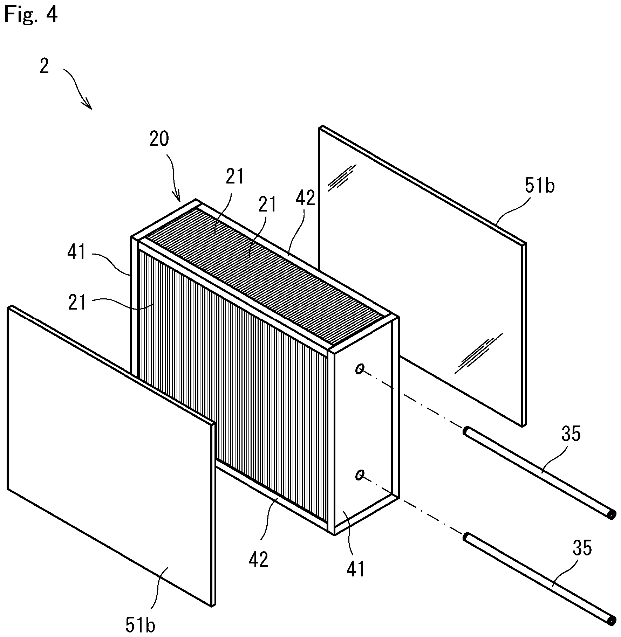

Next, an air cleaning device 2 according to a second embodiment will be described with reference to Fig. 4. The difference from the air cleaning device 1 according to the first embodiment is in the positional relationship that the ultraviolet irradiators 35 have with the thin-plate array unit 20, whereas other components are identical to those of the air cleaning device 1. The identical components will be given the same reference signs, and detailed descriptions thereof will be omitted. Although Fig. 4 only illustrates the thin-plate array unit 20, the ultraviolet irradiators 35, and peripheral components thereof as the relevant part of the air cleaning device 2, the fact that they are accommodated together with the air blower 13, the controller 15, and so on in the casing 10 and that the inlet 11h and the outlet 12 are provided is similar to the air cleaning device 1 shown in Fig. 1.

In the air cleaning device 2, the ultraviolet irradiators 35 are provided to extend through one of the pair of support side surfaces 41 and through at least one or more of the multiple thin plates 21 constituting the thin-plate array unit 20. The pair of support side surfaces 41 are coupled to each other by a pair of first side surfaces 51b that are perpendicular thereto. The space S provided in the air cleaning device 1 is nonexistent between each first side surface 51b and the thin-plate array unit 20. The pair of first side surfaces 51b cover side surfaces parallel to each other in the air flowing direction in the thin-plate array unit 20. The inner surfaces of the pair of first side surfaces 51b are mirror surfaces or mirror-polished surfaces.

Although a case where there are two ultraviolet irradiators 35 is illustrated, the number of ultraviolet irradiators 35 is not limited to this.

The air cleaning device 2 having such a configuration exhibits advantages identical to the advantages described with respect to the air cleaning device 1.

In addition, in the air cleaning device 2, the distance between each ultraviolet irradiator 35 and the coating layers is shorter than that in the air cleaning device 1, so that the efficiency for decomposing organic matters is high, whereby the organic matters can be decomposed within a short period of time. Furthermore, in contrast to the air cleaning device 1 in which the mirror surfaces or the mirror-polished surfaces (i.e., the inner surfaces of the first side surfaces 51) are located at the rear side relative to the ultraviolet irradiation direction, the mirror surfaces or the mirror-polished surfaces (i.e., the inner surfaces of the first side surfaces 51b) in the air cleaning device 2 are surfaces that are directly irradiated with the ultraviolet light. Therefore, the intensity of the ultraviolet light reflected by the mirror surfaces or the mirror-polished surfaces is high, so that the effect of suppressing attenuation of the ultraviolet light after irradiation is higher.

Furthermore, in contrast to the air cleaning device 1 having the space S between the thin-plate array unit 20 and each first side surface 51, the air cleaning device 2 does not have such a space, so that the entire device can be made compact.

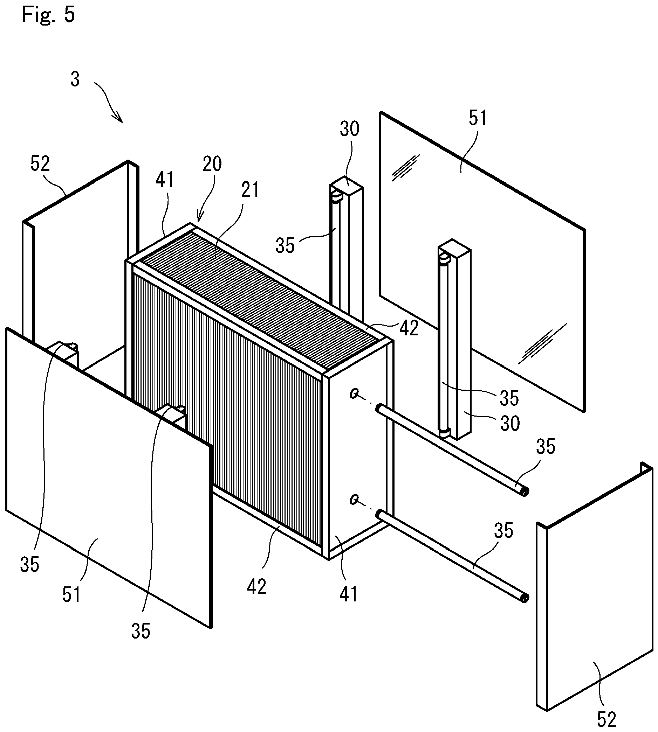

Next, an air cleaning device 3 according to a third embodiment will be described with reference to Fig. 5. The difference from the air cleaning device 1 according to the first embodiment and the air cleaning device 2 according to the second embodiment is in the positional relationship that the ultraviolet irradiators 35 have with the thin-plate array unit 20, whereas other components are identical to those of the air cleaning devices 1 and 2. The identical components will be given the same reference signs, and detailed descriptions thereof will be omitted. Although Fig. 5 only illustrates the thin-plate array unit 20, the ultraviolet irradiators 35, and peripheral components thereof as the relevant part of the air cleaning device 3, the fact that they are accommodated together with the air blower 13, the controller 15, and so on in the casing 10 and that the inlet 11h and the outlet 12 are provided is similar to the air cleaning device 1 shown in Fig. 1.

The positional relationship between the thin-plate array unit 20 and each ultraviolet irradiator 35 in the air cleaning device 3 is equivalent to a combination of the configuration in the first embodiment and the configuration in the second embodiment. Specifically, the air cleaning device 3 has both the ultraviolet irradiators 35 attached to the pair of first side surfaces 51 each having the space S with respect to the thin-plate array unit 20, and the ultraviolet irradiators 35 provided to extend through one of the pair of support side surfaces 41 and through at least one or more of the multiple thin plates 21 constituting the thin-plate array unit 20.

The air cleaning device 3 having such a configuration exhibits advantages identical to the advantages described with respect to the air cleaning device 1.

In addition, in the air cleaning device 3, each coating layer can be irradiated with a larger quantity of ultraviolet light, as compared with the air cleaning devices 1 and 2. Therefore, even when the air contains a large number of organic matters, the organic matters can be decomposed efficiently.

Although the present invention has been described above with reference to preferred embodiments, the present invention is not limited to the above embodiments, and various modifications and design alterations are possible, as indicated below, so long as they do not depart from the scope of the present invention.

For example, in the above description, the air cleaning devices 1, 2, and 3 are used dedicatedly for an air cleaning operation. Alternatively, the thin-plate array unit 20 and each ultraviolet irradiator 35 according to the present invention may be incorporated into an existing air-conditioning and heating device, so that an air cleaning device having both an air-conditioning and heating function and an air cleaning function can be achieved. Since an air-conditioning and heating device is equipped with an inlet, an outlet, and an air blower for causing air to flow from the inlet toward the outlet, these components can be used as-is.

In the above description, each of the air cleaning devices 1, 2, and 3 is equipped with a single thin-plate array unit 20. Alternatively, an air cleaning device having a plurality of thin-plate array units 20 parallel-arranged or series-arranged in the air flowing direction is also possible.

## Claims

1. An air cleaning device comprising:
a thin-plate array unit that has a plurality of thin plates arranged parallel to each other with gaps therebetween and that has a coating layer on each of front and rear surfaces of each thin plate; and
an ultraviolet irradiator that radiates ultraviolet light onto the coating layers,
wherein each coating layer is a metal-substituted hydroxyapatite layer in which a portion of calcium in calcium hydroxyapatite is substituted by metal selected from titanium, zirconium, iron, and tungsten.

2. The air cleaning device according to Claim 1,
wherein each of the thin plates has a shape having a pattern with repetitive recesses and protrusions on the front and rear surfaces, and the recesses and the protrusions are reflected on each coating layer.

3. An air cleaning method using the air cleaning device according to Claim 1, the air cleaning method comprising:
causing air to flow through the gaps between the thin plates; and
using a calcium hydroxyapatite portion in the metal-substituted hydroxyapatite constituting each coating layer to adsorb organic matters contained in the air and decomposing the adsorbed organic matters in accordance with a photocatalytic function by the portion of the metal-substituted hydroxyapatite in which the calcium is substituted by the metal.

4. The air cleaning method using the air cleaning device according to Claim 2, the air cleaning method further comprising:
causing a turbulent flow to occur in the air flowing through the gaps between the thin plates in accordance with the pattern with the repetitive recesses and protrusions to enhance a degree of contact between the air and each coating layer, as compared with when the air is a laminar flow.
